Europäisches Patentamt

European Patent Office

Office européen des brevets

(11) Publication number: **0 064 071**
**B1**

(12) # EUROPEAN PATENT SPECIFICATION

(45) Date of publication of patent specification: **15.05.85**

(51) Int. Cl.⁴: **C 02 F 1/32**

(21) Application number: **81903020.6**

(22) Date of filing: **09.11.81**

(86) International application number:
**PCT/NO81/00040**

(87) International publication number:
**WO 82/01703 27.05.82 Gazette 82/14**

(54) **APPARATUS FOR DISINFECTION OF LIQUIDS.**

(30) Priority: **10.11.80 NO 803371**

(43) Date of publication of application:
**10.11.82 Bulletin 82/45**

(45) Publication of the grant of the patent:
**15.05.85 Bulletin 85/20**

(84) Designated Contracting States:
**FR**

(56) References cited:
**CH-A- 457 302**
**DE-A-2 735 550**
**US-A-3 562 520**
**US-A-3 566 105**
**US-A-4 103 167**
**US-A-4 204 956**

**Patent Abstracts of Japan, abstract of JP
55-157.378, 8 December 1980**

(73) Proprietor: **INTERNATIONAL FARVEFABRIK A/S
Michael Krohnsgatan 59
N-5000 Bergen (NO)**

(72) Inventor: **VALKNER, Magnus
Ibsensgt. 114 B
N-5000 Bergen (NO)**

(74) Representative: **Waxweiler, Jean et al
OFFICE DENNEMEYER S.à.r.l. 21-25 Allée
Scheffer P.O.Box 41
L-2010 Luxembourg (LU)**

Courier Press, Leamington Spa, England.

## Description

The invention relates to an apparatus for disinfecting liquids which comprises a photocell device responsive to ultra violet light from an ultra violet light emitter for irradiating a liquid in an irradiation chamber, and a device for measuring the rate of flow of said liquid through said chamber.

Disinfection by means of ultraviolet (UV) irradiation has been used for 20—30 years. While e.g. in Norway UV-absorption got its breakthrough only in the middle of the seventies, it is now mandatory on board all Norwegian ships above a certain size and is also employed in many waterworks instead of or as a supplement for disinfection by chlorination.

If the water which is to be disinfected contains many particles, for example includes larger amounts of humus particles, it ought to be filtered before it is passed through the irradiation chamber.

In order that the UV-light shall kill at least 99% of pathegonic bacteria, spores and fungus present, these must be exposed to a radiation dose of at least 8,000 µWs/cm². In order to have a certain safety margin, there is required from the side of the Norwegian authorities, represented by SIFF, that is to say the States Institute for Public Health, a radiation dose of at least 16,000 µWs/cm².

The radiation dose D is dependent upon the radiation intensity I and the irradiation time t:

$$D = I \times t.$$

The radiation intensity is dependent upon the effect of the UV-source (which decrease with its period of operation), the distance between the UV-source and the photocell and the purity of the liquid and the irradiation time depends upon the retention period of the liquid in the irradiation chamber.

US—A—4204956 discloses a water purification system comprising a tank through which water is passed and where it is irradiated UV sources emitting in the tank, UV sensors, each sensor being connected to a sensor readout meter from which the radiation level may be visually determined, and a fluid flowmeter which output signal is employed in conjunction with the intensity of radiation and tanek volume to ensure application of the desired UV dosage.

DE—A—2735550 discloses in an apparatus for the disinfection of liquids, the use of the difference between the signal from a tachogenerator and a UV radiation sensor to produce an amplified signal for monitoring a pump responsible for the flow of liquid.

In other known apparatus for the disinfection of liquids, the radiation intensity which is received by the UV-photocell is converted to electrical pulses which are transferred to a meter where the intensity can be measured. However, no measurement is effected of the rate of flow of the liquid or its retention time in the irradiation chamber. Therefore, it is not possible to know what dose the microorganisms are exposed to at any time. This is a serious disadvantage with known apparatus since, as mentioned above, the radiation dose is also dependent upon the irradiation time which is determined by the retention time of the liquid in the chamber and is decisive for the effect of the irradiation.

There is thus a need for an apparatus which makes possible the determination of the radiation dose at any time.

According to the invention an apparatus for disinfecting fluids of the above kind comprises a device which on receiving simultaneous electrical pulses emitted by the flow rate measuring device dependent upon said flow rate and electrical pulses produced by the photocell device dependent on the ultra violet light from the emitter combines said electrical pulses to indicate the momentary radiation dosage.

Conveniently, the cooperating electrical pulses from the flow rate measuring means and the photocell device are indicated as radiation dose and a radiation dosage-indicating device, where they are preferably shown in µWs/cm² (ultrarad). Thereby, it is possible to read off the momentary radiation dose directly on such a device.

According to a convenient embodiment, the flow rate measuring means is adapted, via an alarm device, to close off or choke a valve on not attaining a predetermined dose. In this connection, it can be mentioned that it is required, e.g. from the Norwegian authorities side that the alarm shall be released and thereby a valve closed, if the minimum dose of 16,000 µWs/cm² is not reached. However, an alarm can also be released by a dose which lies above the minimum dose, if this should be required.

With particular particle-containing, for example humus-containing water, it is usual to arrange a filter upstream from the irradiation chamber.

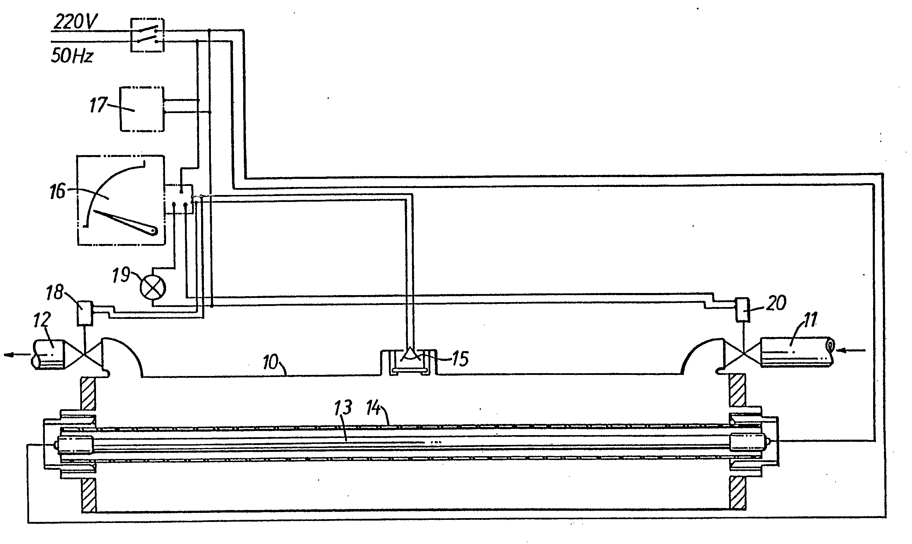

The invention will be further explained in the following description having regard to the accompanying drawing which shows a block diagram of the apparatus according to the invention.

Water or another fluid is led into a cylindrical irradiation chamber 10 through an inlet 11 in the direction shown by the arrow. The water is led out of the chamber through an outlet 12. Inside the chamber, along the axis, there is arranged a UV-light tube 13 which is surrounded by a quartz tube 14, which has good permeability to UV-light (in contrast to conventional glass). The quartz tube 14 is arranged to protect the UV-light tube 13. Rays from the light tube 13 strike a UV-sensitive photocell 15 and are converted to electrical pulses. For recording of the operative time of the light tube 13 there is arranged a time counter 17, since as mentioned the intensity of the tube decreases with its operative time, and it is therefore necessary to effect a replacement of the light tube after a certain number of operative hours, usually about 9,000 hours.

To the outlet of the irradiation chamber 10 there is connected a flow meter 18 which produces in a manner known *per se* electrical pulses dependent upon the speed of flow of the liquid. The pulses from the flow meter are caused to cooperate with the pulses from the UV-photocell 15 and are fed to a meter 16, where they are indicated as a radiation dose, directly in µWs/cm². If a predetermined radiation dose is not reached, a signal is emitted from the meter 16 which releases an alarm in an alarm device 19 which acts to close or throttle a valve 20. If the transmission of UV-rays through the liquid is reduced, for example, as a consequence of poorer water quality with an increased need for irradiation time as a consequence of this, there will thus be effected choking or closing of the valve, until satisfactory conditions are reestablished.

### Claims

1. Apparatus for disinfecting liquids which comprises a photocell device (15) responsive to ultra violet light from an ultra violet light emitter (13) for irradiating a liquid in an irradiation chamber (10), and a device (18) for measuring the rate of flow of said liquid through said chamber (10), characterized in that said apparatus comprises a device (16) which on receiving simultaneous electrical pulses emitted by the flow rate measuring device (18) dependent upon said flow rate and electrical pulses produced by the photocell device (15) dependent on the ultra violet light from the emitter (13) combines said electrical pulses to indicate the momentary radiation dosage.

2. Apparatus according to claim 1, characterised in that said apparatus comprises an alarm device (19) energisable by the radiation dosage-indicating device (16) when said dosage is below a predetermined level to at least partially close a valve (20) for controlling the flow of liquid through the chamber (10).

3. Apparatus according to claim 1 or 2, characterised in that the flow rate measuring device (18) is connected to the outlet (12) of the irradiation chamber (10).

### Patentansprüche

1. Vorrichtung zum Desinfizieren von Flüssigkeiten, mit einer Photozellenvorrichtung (15), welche auf das ultraviolette Licht eines Ultraviolettlichtsenders (13) für die Bestrahlung einer Flüssigkeit in einer Bestrahlungskammer (10) und mit einer Vorrichtung (18) zur Messung der Fliessgeschwindigkeit der Flüssigkeit durch die Kammer (10), dadurch gekennzeichnet, dass die Vorrichtung eine Einrichtung (16) umfasst, welche, beim gleichzeitigen Empfangen elektrischer Impulse, die von der Fliessgeschwindigkeitsmesseinrichtung (18) in Anhängigkeit von der Fliessgeschwindigkeit erzeugt werden und elektrischen Impulsen, welche durch die photozellenvorrichtung (16) erzeugt werden in Abhängigkeit von dem ultravioletten Licht des Senders (13), diese elektrische Impulse kombiniert, um die momentane Strahlungsdosierung anzuzeigen.

2. Vorrichtung nach Anspruch 1, dadurch gekennzeichnet, dass die Vorrichtung eine Alarmvorrichtung (19) umfasst, welche von der Strahlungsdosierungsanzeigeeirichtung (16) betätigt werden kann, wenn die Dosierung unterhalb einem vorgegebenen Niveau liegt, um eine Ventilvorrichtung (20) wenigstens teilweise zu schliessen, um den Durchfluss der Flüssigkeit durch die Kammer (12) zu steuern.

3. Vorrichtung nach Anspruch 1 oder 2, dadurch gekennzeichnet, dass die Fliessgeschwindigkeitsmesseinrichtung (18) an den Ausgang (12) der Bestrahlungskammer (10) angeschlossen ist.

### Revendications

1. Appareil pour désinfector der liquides, comprenant un dispositif (15) à cellule photoélectrique répondant à la lumière ultraviolette d'un émetteur (13) de lumière ultraviolette pour irradier un liquide dans une chambre d'irradiation (10) et un dispositif (18) pour mesurer la vitesse d'écoulement du liquide à travers ladite chambre (10), caractérisé en ce que l'appareil comprend un dispositif (16) qui, suite à la réception simultanée d'impulsions électriques émises par le dispositif (18) de mesure de la vitesse d'écoulement en fonction de la vitesse d'écoulement et d'impulsions électriques produits par le dispositif (15) à cellule photoélectrique en fonction de la lumière ultraviolette de l'émetteur (13), combine ces impulsions électriques pour indiquer le dosage momentané de la radiation.

2. Appareil selon la revendication 1, caractérisé en ce que l'appareil comprend un dispositif d'alarme (19) pouvant être activé par le dispositif (16) indiquant le dosage de radiation quand le dosage est inférieur à un niveau prédéterminé pour fermer au moins partiellement une vanne (20) pour contrôler l'écoulement du liquide à travers la chambre (10).

3. Appareil selon la revendication 1 ou 2, caractérisé en ce que le dispositif (18) de mesure de la vitesse d'écoulement est connecté à la sortie (12) de la chambre d'irradiation (10).